# EUROPEAN PATENT APPLICATION

(11) **EP 2 656 849 A1**
(43) Date of publication of application: **30.10.2013**
(21) Application number: 11857002.7
(22) Date of filing: 23.11.2011
(51) Int. Cl.: A61K 31/704, A61K 9/127, A61K 47/24, A61K 31/4706, A61P 35/00

(54) **LIPOSOME COMPRISING COMBINATION OF CHLOROQUINE AND ADRIAMYCIN AND PREPARATION METHOD THEREOF**

(30) Priority: 27.01.2011 CN 201110025330
(71) Applicant: Zhejiang University, Hangzhou, Zhejiang 310058 (CN)
(72) Inventor: QIU, Liyan, Hangzhou Zhejiang 310058 (CN); YAO, Mingfei, Hangzhou Zhejiang 310058 (CN)
(74) Representative: Gulde Hengelhaupt Ziebig & Schneider
(86) International application number: PCT/CN2011/082684
(87) International publication number: WO 2012/100590

(57) **Abstract**

A liposome and preparation method thereof; the liposome is prepared from drugs, a phospholipid, a cholesterol-based compound, an internal buffering system and a pH adjusting agent; the drugs, the phospholipid and the cholesterol-based compound are in a weight ratio of 1:2-100:1-35; the drugs is a combination of a chloroquine-based drug and an adriamycin-based drug.

## Description

### FILED OF THE INVENTION

The present invention relates to liposomal pharmaceutical technology, and more specifically, to a liposome comprising a combination of chloroquine and doxorubicin co-encapsulated and preparation methods thereof.

### BACKGROUND OF THE INVENTION

During the past 50 years, cancer prevention and treatment have been made significant progress. However, because of the difficulties of early diagnosis and lack of effective strategy to inhibit cancer metastasis, there is still much difficulty to cure cancer. Therefore, looking for a new and effective treatment is really important and urgent.

Doxorubicin (DOX) is a medicine often used in chemo-therapies (Multidrug resistance in cancer; role of ATP-dependent transporter). It was firstly isolated from a Streptomycin by Milan Farotalia research laboratory in early 1960s. It was the second anthracycline antibiotic discovered after daunorubicin. Doxorubicin and daunorubicin have almost the same chemical structure except for a hydroxyl group. However, daunorubicin is only effective for acute leukemia, while doxorubicin is used for broad-spectrum anti-cancer treatment. More than 2,000 doxorubicin analogues have been reportedly tested for anti-cancer effect since the clinical use of doxorubicin was approved by the FDA of the United States in 1974. However, multidrug resistance (MDR) is frequently induced during the administration of DOX in a large amount. Therefore, MDR is a major obstacle to the success of clinical chemotherapy. In order to solve these problems, at present, there are mainly four strategies to reverse MDR: 1) applying MDR reversal agent; 2) chemical modification of the chemotherapy drugs; 3) combining with chemotherapy sensitizer; 4) delivery via nanoparticles and combination the latter two strategies.

Chloroquine is traditionally considered as a safe, effective and inexpensive anti-malarial and anti-rheumatoid medicine. It is also commonly used in gene transfection experiments in order to improve the efficiency of transfection. According to reports of foreign literatures, chloroquine can be used against multiple drug resistance protein 1 (MRP1). Used together with doxorubicin, chloroquine may reduce cell MDR and achieve better anti-cancer results (Reversal of MRP-Mediated Doxorubicin Resistance with Quinoline-Based Drugs). MRP1 belongs to ATP-binding cassette (ABC) family which increases the efflux of a broad class of drugs from cancer cells, resulting in low cancer chemotherapy effect and leading to cell drug resistance (Pharmacogenomics of ABC transporters and its role in cancer chemotherapy). However, earlier research indicated that injection of chloroquine solution causes severe toxicity to normal cells, even death. In addition, doxorubicin has cardiotoxicity and toxicity to normal cells. Reducing dosage is difficult to achieve predictable results. Both chloroquine and doxorubicin have the above problems that need to be addressed.

During the past 20 years, controlled drug release and tumor-targeting drug delivery systems appear as an attractive therapeutic treatment window to treat cell toxins. Among the carriers used, there are nanoparticles, albumin microspheres and liposomes. Liposomes as a chemotherapy drug carrier have previously been re-tested on cows. Since then, liposomes were applied in controlling drug diffusion in order to improve anti-cancer efficiency and reduce the toxicity to normal cells. After drugs are covered or wrapped by liposomes, they can target to the lesion site, which will improve the therapeutic index, reduce the therapeutic dose and ultimately ensure drug safety. So far doxorubicin agents that are approved for clinical use or are under clinical trials are mainly liposomal formulations. For example, DOX liposomes named Myocet have been applied to combination treatment of recurring breast cancer. Although they reduce doxorubicin's toxicity to heart and normal cells, there remains the problems of inducing cell MDR and resulting in unsatisfactory anti-cancer treatment.

### SUMMARY OF THE INVENTION

The present invention relates to a liposome comprising a combination of chloroquine and doxorubicin with a high drug encapsulation effect and wide selection of liposomal carriers. Taking advantage of synergistic effect of chloroquine and doxorubicin, the liposomes are not only used to overcome MRP1 mediated MDR in the cancer treatment, but to reduce the severe toxicity of the two drugs when they are used separately.

The present invention also provides methods to prepare the foregoing liposomes. Doxorubicin and chloroquine are loaded simultaneously into liposomes by pH-gradient method. The preparation process is simple with less control parameters, which reduces production cost and enables production on an industry scale.

### DETAILED DESCRIPTION OF THE INVENTION

A liposome comprising a combination of chloroquine and doxorubicin, wherein said composition comprises drugs, phophatides, a cholesterol derivative, internal buffer systems and a pH-adjusting agent; wherein the weight ratio among drugs, phospholipids and cholesterol derivative is 1: 2~100: 1~35;

Wherein said drugs refer to chloroquine analogues and doxorubicin analogues.

Preferably, said weight ratio among drugs, phospholipids and cholesterol derivative is 1: 10~40: 2.5~10. More preferably, the weight ratio is 1:25:5.

The doxorubicin analogues and chloroquine analogues are the major active pharmaceutical indigredients (API). The proportion of either doxorubicin analogues or chloroquine analogues can be adjusted according to actual needs. The weight ratio of chloroquine analogue to anthracyline analogue is 1~100:1~100 and the preferred is 1:2:1~5.

The chloroquine analogue is a member or several members selected from the group consisting chloroquine or chloroquine in a salt form; wherein said chloroquine in a salt form is one or several members selected from the group consisting chloroquine phosphate, chloroquine hydrochloride, and chloroquine sulfate.

The doxorubicin analogue is a member or several members selected from the group consisting doxorubicin or doxorubicin in a salt form; wherein said doxorubicin in a salt form is doxorubicin hydrochloride.

The phophatide is one or two members selected from natural phospholipids and synthetical phospholipids; wherein said natural phospholipids is soybean phospholipids, lecithin or mixture thereof; wherein said synthetical phospholipids is neutral phospholipids, negatively charged phospholipids or PEG-phospholipids or a mixture thereof; wherein said natural phospholipids is dimyristoyl phosphatidylcholine (DMPC), dipalmitoylphosphatidylcholine (DPPC), distearoylphosphatidylcholine (DSPC), dipalmitoyl phosphatidyl glycerol (DPPG), phosphatidyl ethanolamine (PE), phosphatidyl serine (PS), distearoyl phosphoethanolamine-PEG (DSPE-PEG) or a mixture thereof.

The cholesterol derivative is cholesterol, PEG-modified cholesterol or a mixture of these compounds; wherein said PEG-modified cholesterol is something like polyethylene glycol monomethyl ether cholesterol succinate (CHS-PEG).

The internal buffer systems comprise a citrate buffer, a phosphate buffer or a bicarbonate buffer; the preferred concentration of the citrate buffer is between 0.05mol/L and 0.5mol/L for citrate and sodium citrate. The internal buffer system is helpful to liposome formation, but its amount needs not to be strictly restricted. Normally 5 mL -15 mL is enough for each sample.

The pH-adjusting agent is an alkali or mixed alkalis selected from the group consisting sodiumhydroxide solution, disodium hydrogen phosphate solution, sodium carbonate solution and sodium Bicarbonate solution. The concentration of pH-adjusting agent is 0.05mol/L~0.5mol/L. The terminal exterior pH value of chloroquine and doxorubicin co-encapsulated liposomes in present invention should be regulated within the pH range of 5.5 to 8.0 by pH adjusting agent. Too low pH results in failure to form the concentration gradient and hinders the encapsulation of drugs. However, if the pH is too high, it will cause the liposomes to break. The preferred terminal pH herein is 7.0.

The preparing method of herein said chloroquine and doxorubicin encapsulation of liposomes comprises the following steps:
(1) dissolving the phosphatides and a cholesterol derivative in an organic solvent and adding internal buffer systems to get an emulsion, then evaporating the organic solvent to produce a blank liposomes suspension; Alternatively, dissolving the phosphatides and a cholesterol derivative in an organic solvent and evaporating the organic solvent, then adding internal buffer systems to get a blank liposomes suspension;
(2) dissolving the chloroquine analogue and doxorubicin analogue in the blank liposomes of step (1), adjusting the pH to a value of 5.5-8.0 by the pH-adjusting agent, incubating in water at 30°C~50°C for 10min~60min, and removing free drugs from liposomes to get the chloroquine and doxorubicin loaded liposomes;

In step (1), said organic solvent is diethyl ether or chloroform.

In step (1), after eliminating the organic solvent or after eliminating the organic solvent and adding internal buffer systems, the size of blank liposome suspension can be controlled below 200nm by the method of high pressure homogenization, sonication and micro-jet.

In step (2), the method to move free drugs from liposomes includes Sephadex G-50 gel column filtration or dialysis.

The prepared chloroquine and doxorubicin co-encapsulated liposomes are used as anti-cancer or other related disease injections, which manifested significant anti-cancer cell effect.

The present invention used dynamic light scattering (DLS, Malvern Zetasizer Nano-S90, UK) to measure the size of the liposomes.

The present invention has the following advantages:
1) Various types of phospholipids can be selected including natural lecithin or soybean phospholipids, synthetic neutral phospholipids or negatively charged phospholipids;
2) The formulation achieves high drug encapsulation effect (>90%), small size (<200nm), satisfactory stability and low cost;
3) This formulation can effectively inhibit MRP1 mediated multidrug resistance. The reversal fold is 10 times compared to free doxorubicin and 3 times to doxorubicin liposomes;
4) In the safety range of chloroquine (<20µg/mL), the more proportion of chloroquine encapsulated with doxorubicin in liposomes, the lower is IC₅₀.
5) The synergistic effect of chloroquine and doxorubicin can increase cytotoxicity to cancer cells, and thus reduce the amount of drug used and drug side effects in vivo;
6) Since the PKa of chloroquine and doxorubicin are similar, both at 4.0, the two-step pH gradient method to encapsulate the two simultaneously into liposome used in the present invention is superior, controllable and reproducible.

### EMBODIMENTS OF THE PRESENT INVENTION

The present invention is further described with reference to the specific examples below. It is to be understood that these examples do not limit the scope of the present invention.

### Preparation of blank liposomes

### Example 1

500 mg soybean phospholipids (phosphatidylcholine, purity> 76%) and 100mg cholesterol were dissolved in 20mL diethyl ether. Then, 10mL citric acid - sodium citrate buffer (pH4.0) was added. The mixture was stirred evenly until the mixture became an emulsion. The diethyl ether was evaporated to dryness under reduced pressure. A probe sonicator was used for 5min to obtain about 10mL blank liposome suspension, for further use. The number average size of the blank liposome suspension solution is 129nm.

### Example 2

420mg DPPC, 60mg PE and 100mg cholesterol were dissolved in 10mL diethyl ether and stirred to mix evenly. The solution was placed at the bottom of the round grinding-mouth flask. Evaporating the organic solvent on a rotary evaporator at the temperature of 37°C until a membrane was formed on the bottom. Then 5mL 0.1mol/L citric acid- sodium citrate buffer (pH3.6) was added to the membrane. The mixture was rotated on a rotation evaporator until the liposome membrane solution became milk-white liposonme suspension solution. Finally, the liposomes were sonicated with a probe sonicator for 10min to get smaller particle size to obtain about 10mL blank liposome suspension, for further use. The average size of the blank liposome suspension solution is 133nm.

### Example 3

500mg soybean phospholipids (phosphatidylcholine, purity> 76%) and 40mg cholesterol and 80mg CHS-PEG2000 were dissolved in 20mL diethyl ether. 10mL citric acid - sodium citrate buffer (pH3.6, 0.1mol/L) was added after that. The mixture was stirred evenly until the mixture became an emulsion. The diethyl ether was evaporated to dryness under reduced pressure. A probe sonicator was used for 5min to obtain about 10mL blank liposome suspension, for further use. The number average size of the blank liposome suspension is 137nm.

### Example 4

450mg DSPC, 100mg DPPG and 100mg cholesterol were dissolved in 50mL diethyl ether. 20mL citric acid - sodium citrate buffer (pH3.6, 0.1mol/L) was added after that. The mixture became an emulsion, which was then transferred to a round grinding-mouth flak under 37°C water bath. The diethyl ether was evaporated to dryness under vacuum with a rotary evaporator to obtain milk white liposome suspension. Using micro-jet to get smaller and uniform particle size to obtain about 5mL blank liposome suspension, for further use. The number average size of the final solution is 131nm.

### Example 5

500mg DMPC, 50mg PS and 20mg cholesterol were dissolved in 20mL diethyl ether. 10mL citric acid - sodium citrate buffer (pH3.6) was added after that. The mixture was stirred to mix evenly and became an emulsion. The diethyl ether was evaporated to dryness under reduced pressure. A probe sonicator was used for 5min to obtain about 10mL blank liposome suspension, for further use. The number average size of the blank liposome suspension is 134nm.

### Example 6

420mg DPPC, 60mg PE and 100mg cholesterol were dissolved in 10mL diethyl ether. The solution was placed at the bottom of the round grinding-mouth flak. Evaporating the organic solvent on a rotary evaporator at the temperature of 40°C until a membrane was formed on the bottom. Then 5mL 0.1mol/L citric acid- sodium citrate buffer (pH3.6) was added to the membrane. The mixture was rotated on a rotation evaporator until the liposome membrane solution became milk-white liposome suspension solution. Finally, a probe sonicator was used for 5min to obtain about 10mL blank liposome suspension, for further use. The number average size of the blank liposome suspension is 200nm.

### Example 7

500mg soybean phospholipids (phosphatidylcholine, purity> 76%) and 40mg cholesterol and 80mg CHS-PEG2000 were dissolved in 20mL diethyl ether. 10mL citric acid - sodium citrate buffer (pH3.6, 0.1mol/L) was added after that. The mixture was mixed evenly and became an emulsion. The diethyl ether was evaporated to dryness under reduced pressure. A probe sonicator was used for 5min to obtain about 10mL blank liposome suspension, for further use. The number average size of the blank liposome suspension is 95nm.

### Liposomes Encapsulating Chloroquine and doxorubicin

### Example 8

10.5mg chloroquine and doxorubicin mixture (1:20, w/w) was dissolved in the liposome suspensions as shown in Example 1. The transmembrane pH gradient was implemented by adjusting the external pH to 7.0 with 0.2mol/L disodium hydrogen phosphate (Na₂HPO₄) solution and then incubated in a 40 °C water bath for 20min and cooled to room temperature. Unencapsulated chloroquine and doxorubicin were removed by column separation using SephadexG-50 mini columns. The encapsulation rate or effect (EE)of chloroquine and doxorubicin are respectively 94% and 99%. The loading content (LC) of chloroquine and doxorubicin are 0.08% and 1.6%, respectively.

### Example 9

12mg chloroquine and doxorubicin (1:10, w/w) were dissolved in the liposome suspensions as shown in Example1. The transmembrane pH gradient was implemented by adjusting the external pH to 7.0 with 0.2mol/L Na₂HPO₄ solution and then incubated in a 40°C water bath for 20min and cooled to room temperature. Unencapsulated DOX and CQ were removed by column separation using SephadexG-50 mini columns. The EE of chloroquine and doxorubicin are respectively 95% and 99% .The LC of chloroquine and doxorubicin are 0.3% and 1.6%.

### Example 10

15mg chloroquine and doxorubicin (2:1, w/w) were dissolved in the liposome suspensions as shown in Example1. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using Sephadex-G50 mini columns. The EE of chloroquine and doxorubicin are respectively 93% and 99% .The LC of chloroquine and doxorubicin are 1.5% and 0.8%.

### Example 11

20mg chloroquine and doxorubicin (1:1, w/w) were dissolved in the liposome suspensions as shown in Example1. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The EE of chloroquine and doxorubicin are respectively 96% and 97% .The loading effect of chloroquine and doxorubicin are 1.5% and 1.6%.

### Example 12

30mg chloroquine and doxorubicin hydrochloride (1:2, w/w) were dissolved in the liposome suspensions as shown in Example1. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The EE of chloroquine and doxorubicin are respectively 91% and 94% .The LC of chloroquine and doxorubicin are 1.4% and 3.0%.

### Example 13

18mg chloroquine phosphate and doxorubicin (1:5, w/w) were dissolved in the liposome suspensions as shown in Example1. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The encapsulation effect (EE) of chloroquine and doxorubicin are respectively 94% and 98% .The loading effect of chloroquine and doxorubicin are 0.5% and 1.6%.

### Example 14

30mg chloroquine and doxorubicin·HCL (1:2 w/w) were dissolved in the liposome suspensions as shown in Example2. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The encapsulation effect (EE) of chloroquine and doxorubicin are respectively 91% and 95% .The loading effect of chloroquine and doxorubicin are 1.4% and 3.0%.

### Example 15

20mg chloroquine hydrochloride and doxorubicin (1:1 w/w) were dissolved in the liposome suspensions as shown in Example 3. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The EE of chloroquine and doxorubicin are respectively 95% and 96% .The loading effect of chloroquine and doxorubicin are 1.5% and 1.6%.

### Example 16

20mg chloroquine hydrochloride and doxorubicin (1:1 w/w) were dissolved in the liposome suspensions as shown in Example 5. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The EE of chloroquine and doxorubicin are respectively 94% and 96% .The LC of chloroquine and doxorubicin are 1.5% and 1.6%.

### Example 17

30mg chloroquine and doxorubicin hydrochloride (1:2 w/w) were dissolved in the liposome suspensions as shown in Example 4. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The EE of chloroquine and doxorubicin are respectively 92% and 95% .The LC of chloroquine and doxorubicin are 1.4% and 3.0%.

### Example 18

30mg chloroquine and doxorubicin hydrochloride (1:2 w/w) were dissolved in the liposome suspensions as shown in Example 6. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The EE of chloroquine and doxorubicin are respectively 92% and 95% .The LC of chloroquine and doxorubicin are 1.4% and 3.0%.

### Example 19

30mg chloroquine and doxorubicin (1:2 w/w) were dissolved in the liposome suspensions as shown in Example 7. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine and doxorubicin were removed by column separation using SephadexG-50 mini columns. The EE of chloroquine and doxorubicin are respectively 93% and 96% .The LC of chloroquine and doxorubicin are 1.4% and 3.0%.

### Comparison example 1

10mg doxorubicin hydrochloride was dissolved in the liposome suspensions as shown in Example 1. The transmembrane pH gradient was implemented by adjusting the external pH to 7.0 with 0.2mol/L Na₂HPO₄ solution and then incubated in a 40°C water bath for 20min and cooled to room temperature. Unencapsulated doxorubicin was removed by column separation using SephadexG-50 mini columns. The EE and LC of doxorubicin liposomes (DOXL) are 96% and 4.6%, respectively.

### Comparison example 2

10mg chloroquine phosphate was dissolved in the liposome suspensions as shown in Example 1. The transmembrane pH gradient was implemented by adjusting the external pH to 7.0 with 0.2mol/L Na₂HPO₄ solution and then incubated in a 40°C water bath for 20min and cooled to room temperature. Unencapsulated chloroquine was removed by column separation using SephadexG-50 mini columns. The EE chloroquine and LC of chloroquine liposomes (CQL) are 96% and 1.6%, respectively.

### Cytotoxicity Test

Free doxorubicin (FDOX) aqueous solution as Control 1, free chloroquine (FCQ) as Control 2, various liposomes in Comparison examples and Examples are used separately in a cytotoxicity test by [3-(4,5-dimethylthiazol-2-yl)-2,5-diphenyltetrazoliumbromide] (MTT) colorimetric assay as follows:

Logarithmic growth cancer cells were digested by trypsin, washed by PBS buffer solution (containing 0.05% TWEEN-20 phosphate buffer solution with the pH of 7.4), centrifuged to make a cells suspension solution with a concentration of 1×10⁵/mL. The cells suspension solution was seeded in 96-well plates with 100µL solution per well containing 5000 - 10000 cells. After 24 hours' incubation in 37°C, observation under microscopes revealed that the cells grew integrated with each other and attached to the plate walls. The drug-loaded liposomes and doxorubicin were separately solved in saline solutions. All the solutions, based on the amount of doxorubicin, diluted to different concentrations, which are 0.5 µg/ml, 1 µg/ml, 2 µg/ml, 5 µg/ml, 10 µg/ml, 100 µg/ml, 500 µg/ml, respectively. These different formulations were added 25µl per well in increasing concentrations to the wells of the 96-well plate. The cells were allowed to grow for 24 hours before adding 31.5 µl MTT (5mg/mL) per well. Finally, about 4 hours later, all of the culture medium was removed and the precipitates were dissolved in 200µl dimethyl sulfoxide (DMSO). After incubated in a carbon dioxide incubator for 15 minutes, the absorbance values of each well were measured on an enzyme-labelled meter at the wavelength of 570nm and results were recorded. The above tests were repeated three times for each group and each concentration has four wells.

The 50% inhibitory concentration (IC₅₀) is the drug concentration that inhibits the growth of the half cancer cells: the lower the IC₅₀ values, the greater the cytotoxicity. The IC₅₀ values were calculated by IC₅₀.exe software. In addition, the results here demonstrate that, for the same chloroquine phosphate: doxorubicin, different types of phosphatides have no influence to the IC₅₀.

Table 1 exhibits the IC₅₀ (µg/ml) of formulations (Control 1, Control 2 , liposomal preparation from Comparison example 1, Comparison example 2, Examples 8, 9, 10, 11 and 13 ) to human breast cancer cells (MCF-7) and doxorubicin-resistant human breast cancer MCF-7 (MCF-7/ADR);

Table 2 exhibits the IC₅₀ (µg/ml) of formulations (Control 1, Control 2, liposomal preparation from Comparison example 1, Comparison example 2, Examples 8, 9, 10, 11 and 13 ) to human promyelocytic leukemia cells (HL60) and doxorubicin-resistance human promyelocytic leukemia cell (HL60/ADR);

**Table 1**

| | MCF-7 | MCF-7/ADR |
|---|---|---|
| Control 1 | 0.4 | 15.7 |
| Control 2 | 18.1 | 23.3 |
| Comparison example 1 | 0.4 | 4.5 |
| Comparison example 2 | 9.9 | 10.6 |
| Example 10 | 0.4 | 2.7 |
| Example 11 | 0.4 | 3.3 |
| Example 13 | 0.4 | 4.0 |
| Example 9 | 0.4 | 7.1 |
| Example 8 | 0.4 | 9.8 |

**Table 2**

| | HL60 | HL60/ADR |
|---|---|---|
| Control 1 | 0.4 | 13.6 |
| Control 2 | 17.3 | 11.2 |
| Comparison example 1 | 0.4 | 3.7 |
| Comparison example 2 | 12.0 | 12.2 |
| Example 10 | 0.4 | 0.8 |
| Example 11 | 0.4 | 1.5 |
| Example 13 | 0.4 | 2.4 |
| Example 9 | 0.4 | 5.4 |
| Example 8 | 0.4 | 11.2 |

As shown from Tables 1 and 2, the IC₅₀ values of the free chloroquine (FCQ) (Control 2) and chloroquine liposomes (CQL) (Comparison example 2) are nearly all higher than those of the free doxorubicin (FDOX) (Control 1) and doxorubicin liposomes (DOXL) (Comparison example 1), which indicate that FCQ and CQL are less toxic than FDOX and DOXL. All the examples had inhibitory effects on the MDR cells to a certain extent and such effects increase with the increase of the chloroquine ratio, which indicates that the synergy of doxorubicin and liposomes is great and the effect on HL60/ADR is stronger than the effect on MCF-7/ADR. These findings suggest that this novel DOX and CQ co-encapsulated liposome displays great results to overcome the cell drug resistance, especially MRP1-mediated multidrug resistance.

### Industry Applicability

The present invention is suitable for industrialized application and large scale production.

## Claims

1. A liposome composition encapsulating chloroquine and doxorubicin, wherein said composition comprises drugs, phophatides, a cholesterol derivative, an internal buffer system and a pH-adjusting agent; wherein weight ratio among drugs, phospholipids and the cholesterol derivative is 1: 2~100: 1~35;
wherein said pH-adjusting agent regulates pH of a liposome solution within a range of 5.5 to 8.0;
wherein said drugs are chloroquine analogues and doxorubicin analogues;
wherein said chloroquine analogue is a member or several members selected from the group consisting chloroquine and chloroquine in a salt form;
wherein said doxorubicin analogue is a member or several members selected from the group consisting doxorubicin and doxorubicin in a salt form;
wherein said cholesterol derivative is cholesterol, PEG-modified cholesterol or a mixture of these compounds.

2. The liposome composition encapsulating chloroquine and doxorubicin of claim 1, wherein a weight ratio among drugs, phospholipids and the cholesterol derivative is 1: 10~40: 2.5~10.

3. The liposome composition encapsulating chloroquine and doxorubicin of claim 2, wherein a weight ratio among drugs, phospholipids and cholesterol derivative is 1: 25: 5.

4. The liposome composition encapsulating chloroquine and doxorubicin of claim 1, wherein said chloroquine in a salt form is one or several members selected from the group consisting chloroquine phosphate, chloroquine hydrochloride, and chloroquine sulfate; wherein said doxorubicin in a salt form is doxorubicin hydrochloride.

5. The liposome composition encapsulating chloroquine and doxorubicin of claim 1, wherein said phophatide is one or two members selected from natural phospholipids and synthetical phospholipids; wherein said natural phospholipids is soybean phospholipids, lecithin or mixture thereof; wherein said synthetical phospholipids is neutral phospholipids, negatively charged phospholipids or PEG-phospholipids or a mixture thereof.

6. The liposome composition encapsulating chloroquine and doxorubicin of claim 5, wherein said natural phospholipids is dimyristoyl phosphatidylcholine (DMPC), dipalmitoylphosphatidylcholine (DPPC), distearoylphosphatidylcholine (DSPC), dipalmitoyl phosphatidyl glycerol (DPPG), phosphatidyl ethanolamine (PE), phosphatidyl serine (PS), distearoyl phosphoethanolamine-PEG (DSPE-PEG) or a mixture thereof.

7. The liposome composition encapsulating chloroquine and doxorubicin of claim 1, wherein said internal buffer system is selected from the group comprising citrate buffer, phosphate buffer and bicarbonate buffer; wherein said pH-adjusting agent is an alkali.

8. The liposome composition encapsulating chloroquine and doxorubicin of claim 7, wherein said citrate butter contains citric acid and citrate sodium with the concentration in the range of 0.05 mol/L to 0.5 mol/L; wherein said alkali comprises caustic soda solution, disodium hydrogen phosphate solution, natronite, sodium bicarbonate or a mixture of these solution.

9. The liposome composition encapsulating chloroquine and doxorubicin of claim 1, wherein said weight ratio of chloroquine analogues to doxorubicin analogues is 1~100:1~100.

10. A method to prepare the liposome composition encapsulating chloroquine and doxorubicin of claims 1-9, comprising the following steps:
(1) dissolving the phosphatides and cholesterol derivative in an organic solvent and adding the internal buffer system to obtain an emulsion, then evaporating the organic solvent to produce blank liposomes;
(2) dissolving the chloroquine analogue and doxorubicin analogue in the blank liposomes of step (1), adjusting the pH to a value of 5.5-8.0 by the pH-adjusting agent, incubating in water at 30°C for 10min-60min, and removing free drugs from liposomes to get the chloroquine and doxorubicin loaded liposomes;
